(19) Europäisches Patentamt
European Patent Office
Office européen des brevets

(11) Publication number: 0 147 171
A2

# (12) EUROPEAN PATENT APPLICATION

(21) Application number: 84308908.7

(22) Date of filing: 19.12.84

(51) Int. Cl.⁴: C 07 D 401/12
A 61 K 31/44, A 61 K 9/14

(30) Priority: 28.12.83 JP 245213/83

(43) Date of publication of application:
03.07.85 Bulletin 85/27

(84) Designated Contracting States:
AT BE CH DE FR GB IT LI LU NL SE

(71) Applicant: TOKYO TANABE COMPANY LIMITED
7-3 Nihonbashi-Honcho 2-chome
Chuo-ku Tokyo 103(JP)

(72) Inventor: Masato, Kamibayashi
8-5-306 Higashi 5-chome
Hasuda-shi Saitama 349-01(JP)

(72) Inventor: Shinji, Tsuchiya
1614-1 Oaza-Nakazuma Washimiya-machi
Kitakatsushika-gun Saitama 340-02(JP)

(72) Inventor: Tsutomu, Araki c/o Setagaya's Co. Res. of
Tokyo Tanabe Co., Ltd. 29-1 Shimouma 6-chome
Setagaya-ku Tokyo 154(JP)

(72) Inventor: Susumu, Tsuchiya
9-4-403 Yato-cho 2-chome
Tanashi-shi Tokyo 188(JP)

(74) Representative: Skailes, Humphrey John et al,
Frank B. Dehn & Co. Imperial House 15-19 Kingsway
London WC2B 6UZ(GB)

## (54) Powdery preparation of amorphous dihydropyridine compound.

(57) A powdery preparation of amorphous 1,4-dihydro-2,6-dimethyl-4-(3-nitrophenyl)-3-methoxycarbonylpyridine-5-carboxylic acid 6-(5-phenyl-3-pyrazolyloxy)hexyl ester is provided. The compound has a long-lasting hypotensive effect and the bioavailability of the compound is improved in oral administration by converting the solid structure of the preparation from a crystalline form to an amorphous form.

EP 0 147 171 A2

./...

Croydon Printing Company Ltd.

FIG. 2

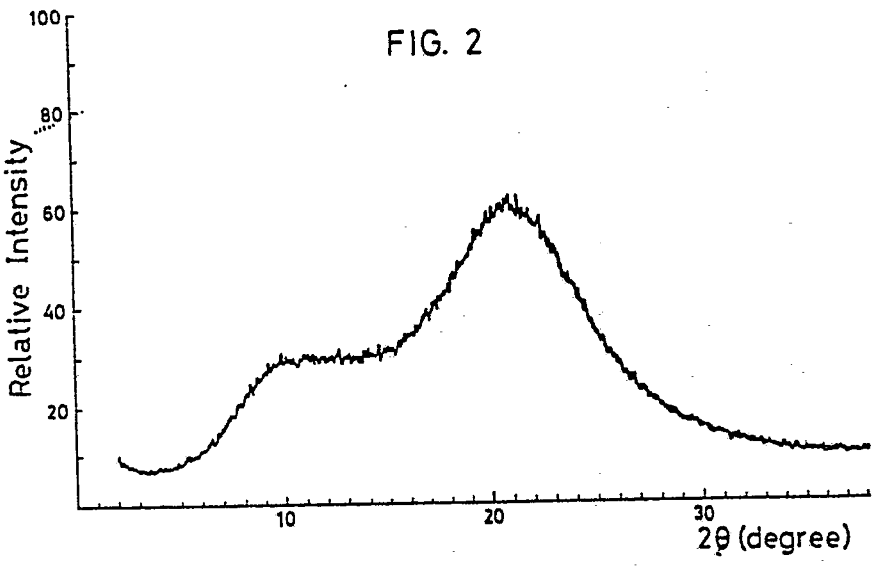

Powdery preparation of amorphous dihydropyridine compound

The present invention relates to a powdered amorphous 1,4-dihydro-2,6-dimethyl-4-(3-nitrophenyl)-3-methoxycarbonylpyridine-5-carboxylic acid 6-(5-phenyl-3-pyrazolyloxy)hexyl ester.

1,4-Dihydro-2,6-dimethyl-4-(3-nitrophenyl)-3-methoxycarbonylpyridine-5-carboxylic acid 6-(5-phenyl-3-pyrazolyloxy)hexyl ester (hereafter tentatively referred to as "dihydropyridine CVA") is a useful drug which possesses a long-lasting hypotensive effect. As its solid structure, a crystalline form (melting point of 151 to 153°C) is known (Japanese Patent Laid-Open 131982/83). However, when a powdery preparation of crystalline dihydropyridine CVA was orally administered, it was found that dihydropyridine CVA was inadequately transferred from the digestive tract into circulating blood, and as a result the hypotensive effect possessed by dihydropyridine CVA per se was not used efficiently. In view of the foregoing circumstances, the present inventors have investigated extensively and have noted that by converting the solid structure from its crystalline form to an amorphous form, bioavailability of dihydropyridine CVA powdery preparation is improved on oral administration and the hypotensive effect is significantly improved.

According to the present invention, a powdered preparation of dihydropyridine CVA characterized by an amorphous solid structure is provided.

For an understanding of the principles of the present invention, reference is made to the following description of typical embodiments thereof as illustrated in the accompanying drawings.

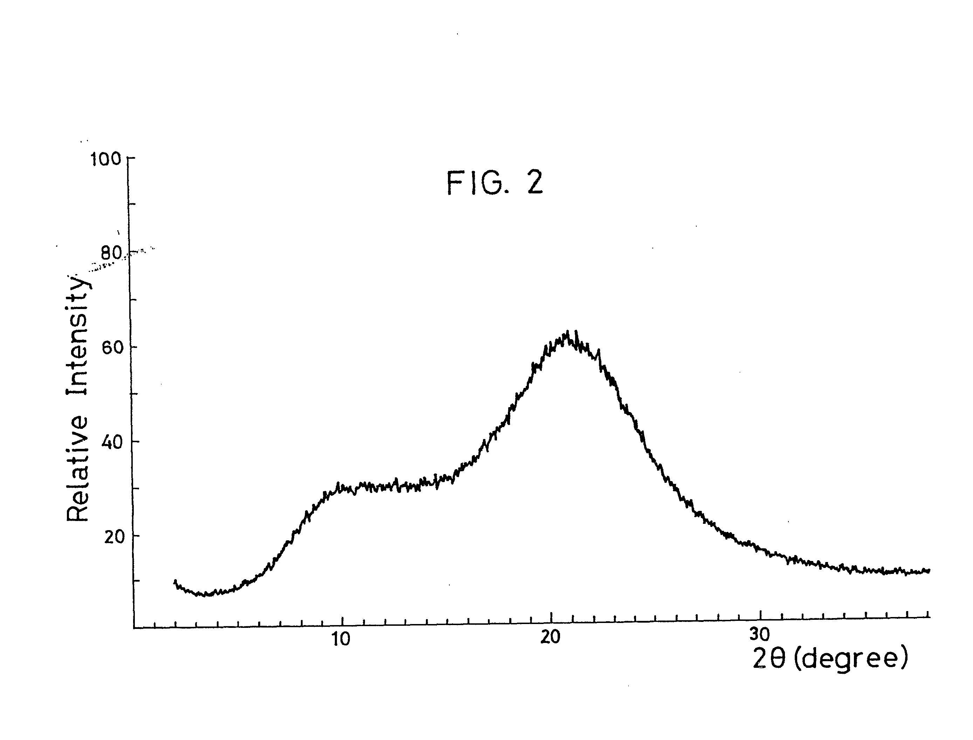

Figs. 1 and 2 show powder x ray diffraction patterns (Cu-Kα) of powder preparations of crystalline and amorphous dihydropyridine CVA, respectively, obtained by measurement using Geiger Flex RAD-rA (manufactured by Rigaku Electric Co., Ltd.).

The powder of the present invention can be prepared by melting pale yellow crystals of dihydropyridine CVA (e.g. prepared in accordance with the process described in Japanese Patent Laid-Open 131982/83) at 155 to 170°C under normal pressure or under reduced pressure, cooling the resulting viscous substance at temperatures lower than 40°C to solidify it and then grinding the solid to a powder (hereafter simply referred to as "melting method"); or by dissolving the crystals in an organic solvent with heating, adding the solution dropwise to water kept at temperatures lower than 10°C, collecting the precipitated powders by filtration and then drying the powders (hereafter simply referred to as "solution method"). In the melting method, the melting is effected within 30 minutes, preferably within 10 minutes. The grinding is carried out using a mortar, a ball mill, a colloid mill, a fluid energy mill, etc., if necessary and desired, under cooling or freezing, in such a manner that a mean particle size of the resulting powder be smaller than 30 µm. In the solution method, suitable examples of organic solvents to be used include methanol, ethanol, isopropanol, acetonitrile and acetone and the amount of the organic solvent to be used is generally 5 to 100 ml per 1 g of crystal. The dissolution may be carried out from 30°C to a boiling point of the solvent used. According to the solution method, powders having a mean particle size exceeding 30 µm are obtained sometimes; in this case, it is desirable to pulverize the powder to obtain a mean particle size smaller than 30 µm.

The thus obtained powder of the present invention may be examined for its hypotensive effect, as described below.

Hypotensive effect:

The test was performed using 5 male spontaneously hypertensive rats (SHR) as one group, weighing 320 to 380 g, warmed for about 5 minutes in a warm box previously kept at 37°C. Powdery preparations of the present invention having mean particle sizes of 3.7 µm and 30 µm were orally given to rats at a dose of 30 mg/kg without anesthesia. Hypotensive effect was determined by measuring systolic blood pressure prior to administration and 2 and 4 hours after administration. The measurement of blood pressure was performed by the tail cuff method, using programmed electro-sphygmomanometer model PE-300 (manufactured by Narco Co., Ltd., USA). The mean particle size of the preparations tested was measured with light transmission type graininess distribution measurement device SKN Model 500 (manufactured by Seishin Enterprise Co., Ltd.). Each preparation tested was suspended in a 0.5 % aqueous solution of carboxymethyl cellulose sodium salt and was given orally. The results are shown in Table 1. In the table, hypotensive effect is expressed by percentage of a difference between a mean of systolic blood pressure prior to administration of the preparations tested and a mean of systolic blood pressure 2 and 4 hours after administration to the former. The hypotensive effect of a powdery preparation (mean particle size, 3.5 μm) of crystalline dihydropyridine CVA determined by the experiment under the same conditions is also shown in the table for purpose of comparison. In the test for hypotensive effect, a significant difference was seen between the group treated with the preparation of the invention and the control group, respectively (below 0.01 at p-value).

Table 1

| Preparation Tested | Mean Particle Size (μm) | Dose (mg/kg) | Systolic Blood Pressure Before Administration (mmHg) | Hypotensive effect (Δ %) 2 Hours After | Hypotensive effect (Δ %) 4 Hours After |
|---|---|---|---|---|---|
| Control Group | - | - | 175 ± 5 | -1 ± 2 | 2 ± 2 |
| Powdery Preparation of This Invention | 30 | 30 | 178 ± 9 | 66 ± 7 | 66 ± 6 |
| | 3.7 | 30 | 177 ± 7 | 68 ± 6 | 67 ± 5 |
| Powdery Preparation of Crystalline Dihydropyridine CVA | 3.5 | 30 | 176 ± 8 | 19 ± 5 | 19 ± 6 |

As is clear from Table 1, the powdery preparations of the present invention exert long-lasting hypotensive effects approximately 3 times higher than that of the powdery preparation of crystalline dihydropyridine CVA without being influenced by particle size of the powder, and it is thus recognized that the powdery preparations of the present invention are antihypertensive drugs which are suited for oral administration. In a test performed using a powdery preparation of the present invention but having a mean particle size exceeding 30 μm, e.g., 100 μm, in a similar manner, hypotensive effect almost equivalent to the results shown in Table 1 was also confirmed.

The powdery preparation of the present invention can be prepared into granules, tablets, pills or capsules in a conventional manner, by appropriately adding thereto excipients, binders or disintegrators, etc.

The present invention will be described more in detail with reference to a reference example and examples below.

Reference Example

Using Jet O Mizer Mill Grinder (manufactured by Seishin Enterprise Co., Ltd.) , 200 g of pale yellow crystals of dihydropyridine CVA was ground into powders to obtain 162 g of a pale yellow powdery preparation having a mean particle size of 3.5 μm. A differential scanning calorimetry curve (hereafter simply referred to as "D.S.C. Curve") of the powdery preparation measured under the condition at a temperature range of 75 to 170°C and a temperature-elevating rate of 8°C/min. showed a endothermic peak at 153°C. Further, the x ray powder diffraction pattern of the preparation was as shown in Fig. 1.

Example 1 (melting method)

In a glass container, 10.00 g of pale yellow crystals of dihydropyridine CVA was heated at 155°C for 5 minutes under reduced pressure to obtain a viscous substance. After the viscous substance was cooled to about 10°C to solidify it, the solid was transferred into a porcelain mortar and ground into powders at room temperature to obtain 9.60 g (yield 96.0 %) of a pale yellow powdery preparation of dihydropyridine CVA. A mean particle size of the thus obtained powdery preparation was 30 μm. This powdery preparation, 2.00 g, was taken into a mortar made of agate and further ground carefully into powder at room temperature to obtain 1.68 g (yield, 84.0 %) of a pale yellow powdery preparation of amorphous dihydropyridine CVA having a mean particle size of 3.7 μm.

D.S.C. Curve (temperature-elevating rate: 8°C/min.):

Neither endothermic nor exothermic peak appeared within a range of 75 to 170°C.

x ray powder diffraction pattern (Cu-Kα):

as shown in Fig. 2

Example 2 (solution method)

To 50 ml of ethanol, 5.00 g of pale yellow crystals of dihydropyridine CVA were added. The mixture was heated under reflux to dissolve the crystals. The resulting solution was added dropwise to 500 ml of ice water with stirring. After completion of the dropwise addition, stirring was continued for further 30 minutes. Then, the precipitated powders were taken out by filtration and thoroughly dried at room temperature under reduced pressure to obtain 4.58 g (yield, 91.6 %) of a pale yellow powdery preparation of amorphous dihydropyridine CVA having a mean particle size of 10 μm.

D.S.C. Curve (temperature-elevating rate: 8°C/min.):

Neither endothermic nor exothermic peak appeared within a range of 75 to 170°C.

x ray powder diffraction pattern (Cu-Kα):

The same behavior as shown in Fig. 2 was noted.

CLAIMS

1. 1,4-Dihydro-2,6-dimethyl-4-(3-nitrophenyl)-3-methoxycarbonylpyridine-5-carboxylic acid 6-(5-phenyl-3-pyrazolyloxy)hexyl ester in a powdered amorphous form.

2. A powder as claimed in claim 1 having a mean particle size of not greater than 30 $m^{-6}$.

3. A pharmaceutical composition containing a powder as claimed in claim 1 or claim 2 together with one or more carriers.

4. A process for the preparation of a powder as claimed in claim 1 which comprises melting a crystalline form of the said compound, cooling the material to form a solid and then grinding the solid to a powder.

5. A process for the preparation of a powder as claimed in claim 1 which comprises precipitating the compound by adding a solution thereof in an organic solvent to water at a temperature of below 10°C.

FIG. 1

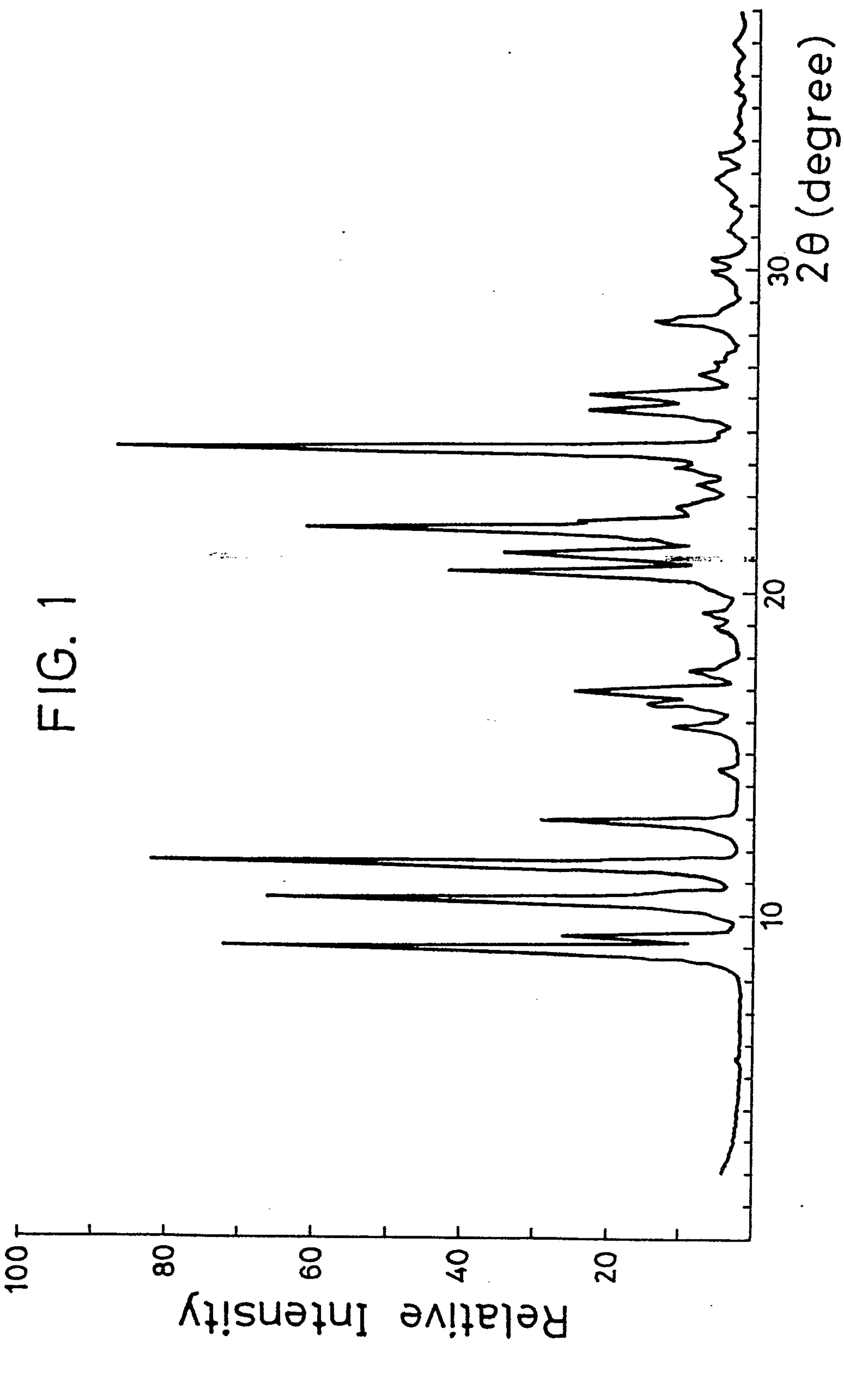

FIG. 2